# EUROPÄISCHE PATENTSCHRIFT

(11) **EP 3 347 510 B1**
(45) Veröffentlichungstag und Bekanntmachung des Hinweises auf die Patenterteilung: **23.02.2022**
(21) Anmeldenummer: 16766900.1
(22) Anmeldetag: 09.09.2016
(51) Int. Cl.: A61Q 19/00

(54) **POLYMERZUSAMMENSETZUNGEN, FASERN UND GARNE MIT PETROLATUM UND/ODER ÖLSÄUREHALTIGEN ÖLEN**
POLYMER FORMULATIONS, FIBRES AND YARN COMPRISING PETROLATUM AND/OR OILS CONTAINING OLEIC ACID
FORMULATIONS POLYMERIQUES, FIBRES ET FILS COMPRENANT DE VASELINE ET/OU DES HUILES AVEC ACIDE OLÉIQUE

(30) Priorität: 11.09.2015 DE 102015217382
(43) Veröffentlichungstag der Anmeldung: 18.07.2018
(73) Patentinhaber: Bauerfeind AG, 07937 Zeulenroda-Triebes (DE)
(72) Erfinder: BAUERFEIND, Hans B., 07937 Zeulenroda-Triebes (DE)
(74) Vertreter: Wohlfahrt, Jan Günther
(86) Internationale Anmeldenummer: PCT/EP2016/071352
(87) Internationale Veröffentlichungsnummer: WO 2017/042362

(56) Entgegenhaltungen:
- WO-A1-2006/007753
- WO-A1-2012/162130
- WO-A1-2015/139965
- CN-C- 100 420 398
- DE-A1-102007 054 702
- GB-A- 190 817 403
- US-A1- 2004 116 018
- US-A1- 2013 053 479
- US-B1- 6 626 961

## Beschreibung

Die vorliegende Erfindung betrifft eine Polymerzusammensetzung, enthaltend ein Polymer und mindestens einen Wirkstoff, Formkörper mit einer solchen Polymerzusammensetzung, die Verwendung der Formkörper und Polymerzusammensetzungen und entsprechende Garne, Vliesstoffe, Kleidungsstücke, Sporthilfsmittel und medizinische Hilfsmittel.

Polymerzusammensetzungen, die durch Additive "funktionalisiert" sind, sind bekannt. So beschreibt die US-A 5,766,746 beispielsweise Cellulosefasern, die eine flammwidrige Komponente enthalten. Die DE 100 37 983 A1 beschreibt Polymerzusammensetzungen mit einem Gehalt an Alkaloid und die DE 100 07 794 A1 Polymerzusammensetzungen mit einem Material aus Meerespflanzen oder -tieren.

Die GB 17403 A offenbart ein Leinengewebe, das mit einer Salbe, enthaltend Vaseline, imprägniert ist.

Die CN 100420398 C offenbart Gesundheitssocken aus Latex und Vaseline.

Die US 2004/116018 A1 offenbart die Herstellung von Fasern aus thermoplastischem Harz und Lipiden und Zusätzen wie Petrolatum.

Die US 2013/053479 A1 offenbart eine Faser aus einem thermoplastischen Polymer und einem Öl.

Die WO 2012/162130 A1 offenbart eine Faser aus einem thermoplastischen Polymer und einem Wachs.

Die DE 10 2007 054 702 A1 offenbart ein Verfahren zur Herstellung cellulosischer Formkörper, wobei die Formköper Wirkstoffe wie Avocadoöl enthalten können.

Die US 6 626 961 B1 offenbart einen nichtgewebten Stoff aus Polyolefin, der mit einer Kombination aus Petrolatum und brasilianischem Nussöl behandelt wird.

Die WO 2006/007753 A1 offenbart ein Polyamidgewebe imprägniert mit Leinsamenöl. Solche Polymerzusammensetzungen werden zu Garnen, textilen Flächen und Kleidungsstücken weiterverarbeitet.

Der Zusatz von Additiven zur Funktionalisierung von Polymerfasern führt oftmals zu Problemen, die bei der Verwendung der Fasern notwendigen Eigenschaften, insbesondere mechanische Festigkeit, Schlingenfestigkeit, Faserdehnung, Scheuereigenschaft und Anfärbbarkeit, dennoch zu erhalten.

Es besteht aber eine stetige Nachfrage nach funktionellen Fasern mit spezifischen Additiven und aus diesen hergestellten Kleidungsstücken, Sporthilfsmittel oder medizinischen Hilfsmitteln, so dass es wichtig ist, solche Fasern bereitzustellen, bei denen die beschriebenen Schwierigkeiten überwunden werden, damit funktionalisierte Fasern und textilen Flächen die entsprechenden Anforderungen an die aus ihnen produzierten Kleidungsstücke, Sporthilfsmittel oder medizinische Hilfsmittel erfüllen können.

Für den Markt besonders relevante Additive sind dabei Additive mit einer Wirkung auf den Organismus des Trägers der aus den Polymerzusammensetzungen hergestellten Kleidungsstücke, Sporthilfsmittel oder medizinische Hilfsmittel.

Bisher bekannte Strukturen beinhalten nur geringe Konzentrationen dieser Stoffe. Diese Stoffe werden durch Tränkung oder Beschichtung auf die Trägermatrix aufgetragen. Dieses führt beispielsweise zu geringen Waschbeständigkeiten, sodass solche Stoffe regelmäßig zur Aufrechterhaltung ihrer Wirkweise neu zugeführt werden müssen.

Daher liegt der vorliegenden Erfindung das technische Problem zugrunde, eine Polymerzusammensetzung bereitzustellen, die ein entsprechendes Additiv enthält, wobei dennoch eine gute Stabilität und Verarbeitbarkeit der aus der Polymerzusammensetzung hergestellten Formkörper resultiert.

Bei eng an der Haut anliegenden Kleidungsstücken und medizinischen Hilfsmitteln, beispielsweise Bandagen und Kompressionsstrümpfen, spielt beim Tragekomfort eine Regulierung der Hautfeuchtigkeit eine Rolle, insbesondere sollte ein Austrocknen der Haut unterbunden werden. Auch sollen solche textile Flächen nicht zu Hautaustrocknung und zu Hautreizungen führen oder bereits bestehende Hauterkrankungen wie atopischer Dermatitis oder Neurodermitis verstärken. Dabei soll auch ein mit solchen Hautkrankheiten verbundener Juckreiz oder Schmerz zumindest verringert werden.

Das der vorliegenden Erfindung zugrunde liegende technische Problem ist auch die Bereitstellung von insbesondere in dieser Hinsicht verbesserten Kleidungsstücken und medizinischen Hilfsmitteln und deren Ausgangsmaterial.

Die vorliegende Erfindung löst das ihr zugrunde liegende technische Problem durch den Gegenstand der unabhängigen Ansprüche.

Insbesondere löst die vorliegende Erfindung das ihr zugrunde liegende technische Problem auch durch ein Garn oder eine textile Fläche, enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl oder einem Fett, wobei das Öl oder das Fett mindestens 25 Gew.-% Ölsäure enthält, einem nicht verseifbaren Lipid aus einem Öl, wobei das Öl mindestens 25 Gew.-% Ölsäure enthält, und Mischungen davon, nach Anspruch 1.

Insbesondere löst die vorliegende Erfindung das ihr zugrunde liegende technische Problem auch durch ein Garn oder eine textile Fläche, enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl, wobei das Öl mindestens 25 Gew.-% Ölsäure enthält, einem nicht verseifbaren Lipid aus einem Öl, wobei das Öl mindestens 25 Gew.-% Ölsäure enthält, und Mischungen davon, nach Anspruch 1.

Insbesondere löst die vorliegende Erfindung das ihr zugrunde liegende technische Problem auch durch ein Garn oder eine textile Fläche, enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl, wobei das Öl mindestens 25 Gew.-% Ölsäure enthält, und Mischungen davon, nach Anspruch 1.

Insbesondere löst die vorliegende Erfindung das ihr zugrunde liegende technische Problem auch durch ein Garn, enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl, wobei das Öl mindestens 25 Gew.-% Ölsäure enthält, und Mischungen davon, nach Anspruch 1.

Insbesondere löst die vorliegende Erfindung das ihr zugrunde liegende technische Problem auch durch eine textile Fläche, enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl, wobei das Öl mindestens 25 Gew.-% Ölsäure enthält, und Mischungen davon, nach Anspruch 1.

Bevorzugt löst die vorliegende Erfindung das ihr zugrunde liegende technische Problem durch Garn oder eine textile Fläche, enthaltend Fasern, wobei die Fasern eine Polymerzusammensetzung umfassen, enthaltend ein Polymer und Petrolatum, nach Anspruch 1.

Petrolatum ist ein Paraffingemisch, das insbesondere aus C12 bis C85 Kohlenwasserstoffen gebildet, insbesondere mit einem überwiegenden Anteil an kristallinen und flüssigen gesättigten Kohlenwasserstoffen mit mehr als 25 Kohlenstoffatomen. Petrolatum hat einen Schmelzpunkt zwischen 25 °C und 60°C, insbesondere 36°C bis 60°C, bevorzugt 38°C und 60°C hat. Petrolatum wurde 1859 entdeckt. Petrolatum ist ein bei Raum- oder Körpertemperatur halbfestes Paraffin.

Petrolatum ist als "Penetrations Enhancer" bekannt. Es verstärkt die Durchdringung des Stratum corneum mit lipophilen Stoffen. Petrolatum bewirkt dort eine Konfigurationsänderung in die orthorhombische Ordnung der interzellulären Lipide und wirkt dem Wasserverlust entgegen. Petrolatum verursacht dabei bei den eingebetteten Corneocyten ein Anschwellen durch die Wasser-Zurückhaltung. Der intrazelluläre Weg ist wesentlich geringer ausgeprägt.

Übliche Stoffe zur Penetrationsverstärkung, beispielsweise Amine, Alkohole, Azone usw, führen zur Disruption des Stratum corneum, um dem zugefügten Wirkstoff, beispielsweise Arzneimittel, Zutritt zu der Dermis zu verschaffen.

Es zeigte sich nun, das Petrolatum, auch in einer Polymerzusammensetzung, sich in vorteilhafter Weise für den hautschonenden Einsatz als Penetrationsverstärker eignet, da es biomimetische Eigenschaften aufweist und wie ein physiologisches Lipid mit den interzellulären Lipiden des Stratum corneum interagiert.

Darüber hinaus ist Petrolatum in vorteilhafter Weise fähig, die Wiederherstellung der Barrierefunktion nach Beschädigung des Stratum corneum zu bewirken. Obwohl Petrolatum chemisch mit Lipiden der Haut nicht verwandt, kann die Mischung gesättigter geradkettiger und verzweigter Kohlenwasserstoffe des Petrolatum im Stratum corneum die Störung im Bereich der interzellulären Lipide verringern oder gar beseitigen.

Auch hat Petrolatum ein vorteilhaftes Spreitungsverhalten.

Es zeigte sich nun, dass sich diese Eigenschaften von Petrolatum auch in Polymerzusammensetzungen und daraus gebildeten Fasern und diese Fasern enthaltenden Garne, textile Flächen, Kleidungsstücke, Sporthilfsmittel oder medizinische Hilfsmittel zu Nutze machen lassen.

Bevorzugt ist daher der mindestens eine Wirkstoff Petrolatum.

Offenbart ist auch eine Polymerzusammensetzung, enthaltend ein Polymer und ein Öl, wobei das Öl mindestens 25 Gew.-% Ölsäure enthält.

Alternativ löst die vorliegende Erfindung das ihr zugrunde liegende technische Problem auch durch ein Garn oder eine textile Fläche, enthaltend Fasern, wobei die Fasern eine Polymerzusammensetzung umfassen, enthaltend ein Polymer und ein Öl, wobei das Öl mindestens 25 Gew.-% Ölsäure enthält, nach Anspruch 1.

Es zeigte sich überraschender Weise, dass sich auch Öle mit einem hohen Ölsäureanteil als Penetrationsverstärker eignen und dies auch als Wirkstoff in einer Polymerzusammensetzung.

Bevorzugt ist eine Polymerzusammensetzung, enthaltend ein Polymer und eine Mischung aus Petrolatum und einem Öl, wobei das Öl mindestens 25 Gew.-% Ölsäure enthält.

Die Erfindung betrifft somit bevorzugt auch ein erfindungsgemäßes Garn oder eine erfindungsgemäße textile Fläche, wobei die Polymerzusammensetzung Petrolatum und das mindestens 25 Gew.-% Ölsäure enthaltende Öl enthält, nach Anspruch 1.

Bevorzugt dient der mindestens eine Wirkstoff zur Penetrationsverstärkung durch die Haut.

Bevorzugt ist also ein erfindungsgemäßes Garn oder eine erfindungsgemäße textile Fläche, wobei der mindestens eine Wirkstoff zur Penetrationsverstärkung durch die Haut dient.

Bevorzugt ist der mindestens eine Wirkstoff, insbesondere das Petrolatum, ein eine Okklusion erzeugender Wirkstoff. Petrolatum ist in vorteilhafter Weise als okklusionserzeugender Wirkstoff geeignet. Auch Sheabutter kann als okklusionserzeugender Wirkstoff verwendet werden, insbesondere in Kombination mit Petrolatum.

Bevorzugt ist also ein erfindungsgemäßes Garn oder eine erfindungsgemäße textile Fläche, wobei der mindestens eine Wirkstoff ein eine Okklusion erzeugender Wirkstoff ist.

Im Zusammenhang mit der vorliegenden Erfindung wird unter Okklusion die Verhinderung oder Reduzierung von Wasserverlust durch die Haut an die Umgebung, wie beispielsweise einer textilen Auflage, verstanden.

Neben weiteren wichtigen Funktionen schützt die menschliche Haut vor Angriffen aus der Umwelt. Zur Aufrechterhaltung ihrer Schutzfunktion ist es von wesentlicher Bedeutung, den Verlust von Wasser aus den tieferen Hautschichten zu vermeiden.

Der Aufbau der menschlichen Haut gliedert sich in drei Bestandteile, nämlich die Oberhaut (Epidermis), die Lederhaut (Dermis) und die Unterhaut (Subcutis). Eine besondere Rolle für den Wassergehalt der Haut spielen die äußeren Schichten des Stratum corneum der Epidermis mit ihren Keratinfilamenten. So kann das Keratinfasergerüst beträchtliche Wassermengen halten. Ziel- und Angriffspunkt der äußeren okklusiven Wirkung eines entsprechenden Stoffes ist die Epidermis. Durch Aufbringen eines solchen Stoffes auf die Hautoberfläche entfaltet sich die Hauptwirkung, nämlich die Unterdrückung eines Entweichens von Wasser (trans-epidermaler Wasserverlust) aus dem Stratum corneum. Durch diese Stoffe wird ein Film gebildet, der das Entweichen von Feuchtigkeit verhindert. Bei der äußeren Okklusion kann somit Wasser aus den tieferen Schichten nachrücken und das Keratinfasergerüst der äußeren Hautschicht mit Feuchtigkeit auffüllen. Dadurch schützt das Stratum corneum vor Austrocknung. Elastizität und Geschmeidigkeit der Haut werden erhalten und die Haut erhält ihr physiologisches Gleichgewicht zum Schutz beispielsweise vor nachteiligen äußeren Einflüssen. Diese okklusive Wirkweise eines Wirkstoffes wird auch als äußere Okklusion bezeichnet.

Die innere Okklusion bezeichnet eine Änderung der Hautlipide-Konfiguration im Stratum corneum. In der hexagonalen Konfiguration können die Kohlenwasserstoffketten der Lipide frei um ihre Achse rotieren. Diese Phase der "lockeren Packung" schafft eine durchlässigere, also permeable Struktur und erleichtert eine unerwünschte Wasserabgabe unter Stress im Stratum corneum mit entsprechenden nachteiligen Folgen, wie beispielsweise Hauttrockenheit. Durch die Einwirkung bestimmter Wirkstoffe wird bei der inneren Okklusion eine dichtere orthorhombische Packung der Lipide erzeugt. Diese Anordnung führt zu einer festeren Struktur. Solch eine molekulare Aktion bewirkt durch die "orthorhombische Packung" der Lipide eine wesentlich geringere Durchlässigkeit der Hautbarriere und reduziert somit signifikant den Wasserverlust aus dem Stratum corneum. Petrolatum als bevorzugter Wirkstoff ist in vorteilhafter Weise ein Durchgangsmittler bei dem Prozess der inneren Okklusion.

Bevorzugt ist der mindestens eine Wirkstoff, insbesondere das Petrolatum, ein eine innere Okklusion erzeugender Wirkstoff.

Bevorzugt ist der mindestens eine Wirkstoff Petrolatum. Bevorzugt ist der Wirkstoff Petrolatum.

Bevorzugt ist der Wirkstoff ein Petrolatum gemäß CAS Nummer 8009-03-8.

Bevorzugt handelt es sich bei dem Petrolatum um Petrolatum USP, also ein Petrolatum, das den USP (US Pharmacopeial Convention) Anforderungen ("USP verified") genügt.

Bevorzugt enthält das Petrolatum höchstens 20 Gew.-% gesättigte, geradkettige Kohlenwasserstoffe mit 20 bis 45 Kohlenstoffatomen. Bevorzugt enthält das Petrolatum mindestens 20 Gew.-% und höchstens 25 Gew.-% gesättigte, geradkettige Kohlenwasserstoffe mit 30 bis 90 Kohlenstoffatomen. Bevorzugt enthält das Petrolatum mindestens 40 Gew.-% gesättigte, geradkettige Kohlenwasserstoffe mit 10 bis 50 Kohlenstoffatomen. Solche Petrolatum-Arten erweisen sich in vorteilhafter Weise als weniger schmierig ("greasy") und spreiten günstig bei Einwirkung von austretenden anderen Wirkstoffen wie Dicapryllyl Carbonat aus einer Faser, die aus der erfindungsgemäßen Polymerzusammensetzung gebildet ist, im Hautbereich.

Bevorzugt enthält die Polymerzusammensetzung, das Garn oder die textile Fläche zusätzlich mindestens einen Wirkstoff, wobei es sich bei dem Wirkstoff um einen eine Okklusion erzeugenden Wirkstoff, insbesondere einen eine innere Okklusion erzeugenden Wirkstoff oder einen eine äußere Okklusion erzeugenden Wirkstoff, um einen feuchtigkeitsspendenden Wirkstoff, oder um einen Schmerz oder Juckreiz reduzierenden Wirkstoff oder Mischungen hiervon handeln kann. Bevorzugt ist der zusätzliche Wirkstoff ein eine Okklusion erzeugender Wirkstoff, insbesondere ein eine innere Okklusion erzeugender Wirkstoff oder ein eine äußere Okklusion erzeugender Wirkstoff.

Im Zusammenhang mit der vorliegenden Erfindung wird unter Okklusion die Verhinderung oder Reduzierung von Wasserverlust durch die Haut an die Umgebung, wie beispielsweise einer textilen Fläche, verstanden.

Bevorzugt ist daher auch ein erfindungsgemäßes Garn oder eine erfindungsgemäße textile Fläche, wobei die Polymerzusammensetzung zusätzlich einen Wirkstoff enthält, ausgewählt aus der Gruppe, bestehend aus Dicaprylyl Carbonat, Cetylisononanoat, Cetearyl Isononanoat, Isopropyl Isostearat, Isostearyl Isostearat, Isopropyl Palmitat, Sheabutter und Mischungen davon.

Erfindungsgemäß ist ein Garn oder eine textile Fläche, wobei die Polymerzusammensetzung zusätzlich einen Wirkstoff enthält, ausgewählt aus der Gruppe, bestehend aus Cetylisononanoat, Cetearyl Isononanoat, Dicaprylyl Carbonat, Isopropyl Isostearat, Isostearyl Isostearat, Isopropyl Palmitat und Mischungen davon.

Cetylisononanoat hat die CAS-Nummer 84878-33-1. Cetearyl Isononanoat hat die CAS-Nummer 11937-03-02.

Bevorzugt ist daher auch ein erfindungsgemäßes Garn oder eine erfindungsgemäße textile Fläche, wobei die Polymerzusammensetzung zusätzlich Cetylisononanoat enthält.

Bevorzugt ist daher auch ein erfindungsgemäßes Garn oder eine erfindungsgemäße textile Fläche, wobei die Polymerzusammensetzung zusätzlich Cetearyl Isononanoat enthält.

Bevorzugt ist daher auch ein erfindungsgemäßes Garn oder eine erfindungsgemäße textile Fläche, wobei die Polymerzusammensetzung zusätzlich Dicaprylyl Carbonat enthält.

Bevorzugt ist daher auch ein erfindungsgemäßes Garn oder eine erfindungsgemäße textile Fläche, wobei die Polymerzusammensetzung zusätzlich Isopropyl Isostearat enthält.

Bevorzugt ist daher auch ein erfindungsgemäßes Garn oder eine erfindungsgemäße textile Fläche, wobei die Polymerzusammensetzung Isostearyl Isostearat enthält.

Bevorzugt ist daher auch ein erfindungsgemäßes Garn oder eine erfindungsgemäße textile Fläche, wobei die Polymerzusammensetzung zusätzlich Isopropyl Palmitat enthält.

Bevorzugt ist daher auch ein erfindungsgemäßes Garn oder eine erfindungsgemäße textile Fläche, wobei die Polymerzusammensetzung zusätzlich Sheabutter enthält.

Bevorzugt ist eine Polymerzusammensetzung, enthaltend ein Polymer, Petrolatum (PET) und Cetylisononanoat (CEIN).

Bevorzugt ist eine Polymerzusammensetzung, enthaltend ein Polymer und eine Mischung aus Petrolatum (PET) und Cetylisononanoat (CEIN).

Alternativ ist eine Polymerzusammensetzung, enthaltend ein Polymer und eine Mischung aus Petrolatum (PET) und Cetearyl Isononanoat. Alternativ ist eine Polymerzusammensetzung, enthaltend ein Polymer und eine Mischung aus Petrolatum (PET) und Dicaprylyl Carbonat (DICA). Dicaprylyl Carbonat stellt den günstigen funktionellen Partner für die lipophile Komponente PET dar und verhindert deren "schmierigen" Effekt ("greasy skin feeling") auf der Haut.

Alternativ ist eine Polymerzusammensetzung, enthaltend ein Polymer und eine Mischung aus Petrolatum und einem Fettsäurederivat, insbesondere Isopropyl Isostearat (IPIS) oder Isostearyl Isostearate (ISIS), die als eine innere Okklusion erzeugende Wirkstoffe bekannt sind.

Bevorzugt ist eine Polymerzusammensetzung, enthaltend ein Polymer und eine Mischung aus Petrolatum und Isopropyl Isostearat. Alternativ bevorzugt ist eine Polymerzusammensetzung, enthaltend ein Polymer und eine Mischung aus Petrolatum und Isostearyl Isostearat.

Petrolatum unterstützt als Penetrationsverstärker die Wirkung von ISIS und IPIS als innere Okklusionsbildner. Darüber hinaus ist Petrolatum selbst ein Wirkstoff der eine Okklusion erzeugt.

Bevorzugt ist daher auch ein erfindungsgemäßes Garn oder eine erfindungsgemäße textile Fläche, wobei die Polymerzusammensetzung Petrolatum und einen weiteren Wirkstoff enthält, ausgewählt aus der Gruppe bestehend aus Cetylisononanoat, Dicaprylyl Carbonat, Isopropyl Isostearat, Isostearyl Isostearat und Mischungen davon.

Bevorzugt ist daher auch ein erfindungsgemäßes Garn oder eine erfindungsgemäße textile Fläche, wobei die Polymerzusammensetzung Petrolatum und einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist eine Polymerzusammensetzung, enthaltend ein Polymer und eine Mischung aus Petrolatum und mindestens einem Öl und/oder eine Mischung aus Petrolatum und mindestens einem Fett.

Bevorzugt ist eine Polymerzusammensetzung, enthaltend ein Polymer und eine Mischung aus Petrolatum und einem Öl und/oder eine Mischung aus Petrolatum und einem Fett.

Bevorzugt ist eine Polymerzusammensetzung, enthaltend ein Polymer und eine Mischung aus Petrolatum und einem Fett. Bevorzugt ist das Fett ein Pflanzenfett.

Bevorzugt enthält das Fett Ölsäure. Bevorzugt enthält das Fett mindestens 25 Gew.-% Ölsäure. Bevorzugt enthält das Fett mindestens 35 Gew.-% Ölsäure. Bevorzugt enthält das Fett mindestens 40 Gew.-% Ölsäure. Bevorzugt enthält das Fett Palmitinsäure. Bevorzugt enthält das Fett mindestens 2 Gew.-% Palmitinsäure. Bevorzugt enthält das Fett Stearinsäure. Bevorzugt enthält das Fett mindestens 30 Gew.-% Stearinsäure. Bevorzugt enthält das Fett Ölsäure, Palmitinsäure und Stearinsäure.

Bevorzugt ist das Fett Sheabutter. Bevorzugt ist somit eine Polymerzusammensetzung, enthaltend ein Polymer und eine Mischung aus Petrolatum und Sheabutter.

Sheabutter wird aus der Karitenuss des Karitebaums gewonnen. Der Karitebaum, auch Sheanussbaum, Schibutterbaum oder Afrikanischer Butterbaum genannt (Vitellaria paradoxa, Syn.: Butyrospermum parkii), ist die einzige Pflanzenart der Gattung Vitellaria in der Familie der Sapotengewächse. Das Besondere an der Sheabutter ist der hohe Anteil an nicht verseifbaren Bestandteilen (etwa 75 % Triterpene, daneben Ölsäure, Triterpenalkohole, Vitamin E, Beta-Karotin und Allantoin). Sheabutter enthält hauptsächlich langkettige, ungesättigte Fettsäuren. Hauptbestandteile sind 40-55 % Ölsäure, 35-45 %Stearinsäure, 3-8 % Linolensäure und 3-7 % Palmitinsäure.

Die in Sheabutter enthaltenen Triterpene haben in vorteilhafter Weise eine antientzündliche Wirkung. Eine Mischung aus Petrolatum USP und Sheabutter ist beispielsweise als Shea XP^{™} der Firma Sonneborn LLC, USA erhältlich. Die Triterpene der Sheabutter können synergistisch mit dem Petrolatum bei der Verbesserung der Hautfeuchte und Verminderung des transepidermalen Wasserverlusts agieren.

Bevorzugt ist eine Polymerzusammensetzung, enthaltend ein Polymer, Petrolatum (PET), Sheabutter und Cetylisononanoat (CEIN).

Alternativ löst die vorliegende Erfindung das ihr zugrunde liegende technische Problem durch eine Polymerzusammensetzung, enthaltend ein Polymer und Sheabutter.

Bevorzugt ist eine Polymerzusammensetzung, enthaltend ein Polymer und eine Mischung aus Petrolatum und einem Öl. Bevorzugt enthält das Öl Ölsäure. Bevorzugt enthält das Öl mindestens 25 Gew.-% Ölsäure. Bevorzugt enthält das Öl mindestens 35 Gew.-% Ölsäure. Bevorzugt enthält das Öl mindestens 40 Gew.-% Ölsäure. Bevorzugt enthält das Öl Palmitinsäure. Bevorzugt enthält das Öl mindestens 20 Gew.-% Palmitinsäure. Bevorzugt enthält das Öl Stearinsäure. Bevorzugt enthält das Öl mindestens 5 Gew.-% Stearinsäure. Bevorzugt enthält das Öl Ölsäure, Palmitinsäure und Stearinsäure.

Alternativ löst die vorliegende Erfindung das ihr zugrunde liegende technische Problem durch eine Polymerzusammensetzung, enthaltend ein Polymer und ein Öl, wobei das Öl mindestens 25 Gew.-% Ölsäure enthält.

Alternativ löst die vorliegende Erfindung das ihr zugrunde liegende technische Problem auch durch ein Garn oder eine textile Fläche, enthaltend Fasern, wobei die Fasern eine Polymerzusammensetzung umfassen, enthaltend ein Polymer und ein Öl, wobei das Öl mindestens 25 Gew.-% Ölsäure enthält.

Bevorzugt ist daher auch ein erfindungsgemäßes Garn oder eine erfindungsgemäße textile Fläche, wobei das Öl mindestens 35 Gew.-% Ölsäure, mindestens 20 Gew.-% Palmitinsäure, mindestens 3 Gew.-% Palmitoleinsäure, mindestens 5 Gew.-% Linolsäuresäure, mindestens 5 Gew.-% Stearinsäuresäure und mindestens 0,5 Gew.-% cis-9-Octadecensäure enthält.

Das Öl kann ein synthetisches oder ein natürliches Öl sein.

Bevorzugt enthält das Öl mindestens 35 Gew.-% Ölsäure. Bevorzugt enthält das Öl mindestens 40 Gew.-% Ölsäure.

Bevorzugt enthält das Öl mindestens 35 Gew.-% Ölsäure und mindestens 20 Gew.-% Palmitinsäure.

Bevorzugt enthält das Öl mindestens 35 Gew.-% Ölsäure, mindestens 20 Gew.-% Palmitinsäure, und mindestens 5 Gew.-% Stearinsäuresäure.

Bevorzugt enthält das Öl mindestens 35 Gew.-% Ölsäure, und zusätzlich Palmitinsäure, Linolsäure und Stearinsäuresäure.

Bevorzugt enthält das Öl mindestens 35 Gew.-% Ölsäure, und zusätzlich Palmitinsäure, Palmitoleinsäure, Linolsäure und Stearinsäuresäure.

Bevorzugt enthält das Öl mindestens 35 Gew.-% Ölsäure, und zusätzlich Palmitinsäure, Palmitoleinsäure, Linolsäure, Stearinsäuresäure und cis-9-Octadecensäure.

Bevorzugt enthält das Öl mindestens 35 Gew.-% Ölsäure, mindestens 20 Gew.-% Palmitinsäure, mindestens 3 Gew.-% Palmitoleinsäure, mindestens 5 Gew.-% Linolsäuresäure, mindestens 5 Gew.-% Stearinsäuresäure und mindestens 0,5 Gew.-% cis-9-Octadecensäure.

Bevorzugt ist daher auch ein erfindungsgemäßes Garn oder eine erfindungsgemäße textile Fläche, wobei das Öl Marula-Öl, Olivenöl und/oder Rapsöl oder ein nicht verseifbares Lipid des Rapsöls ist.

Bevorzugt ist daher auch ein erfindungsgemäßes Garn oder eine erfindungsgemäße textile Fläche, wobei das Öl Marula-Öl, Olivenöl und/oder Rapsöl ist. Die Öle können einzeln oder in Mischungen enthalten sein.

Bevorzugt ist das Öl Rapsöl, insbesondere Canola-Öl. Bevorzugt ist das Öl Canola-Öl. Canola-Öl ist Öl einer neugezüchteten Raps-Varietät, die arm an Erucasäure und Glucosinolat ist. Es wurde gezeigt, dass Canola-Öl sich sehr gut zur Pflege der Haut und/oder zur Verhinderung des Austrocknens der Haut eignet.

Alternativ ist das Öl Marula-Öl. Marula-Öl ist das Öl, das aus den Samen der Frucht des Marula-Baums (*Sclerocarya birrea*) gewonnen wird. Es enthält in vorteilhafter Weise bis zu mehr als 70 gew.-% Ölsäure.

Bevorzugt enthält die Polymerzusammensetzung Petrolatum und Raps-Öl, insbesondere Canola-Öl.

Bevorzugt enthält die Polymerzusammensetzung Petrolatum und Marula-Öl.

Alternativ kann das Öl Olivenöl sein.

Bevorzugt ist auch ein erfindungsgemäßes Garn oder eine erfindungsgemäße textile Fläche, wobei die Polymerzusammensetzung nicht verseifbare Lipide enthält. Die nicht verseifbaren Lipide können alternativ zu dem Öl oder zusätzlich zu dem Öl eingesetzt werden. Bevorzugt stammen die verseifbaren Lipide aus einem Öl, wobei das Öl mindestens 25 Gew.-% Ölsäure enthält. Bevorzugt stammen die nicht verseifbaren Lipide aus Raps-Öl, Olivenöl und/oder Marula-Öl, insbesondere Rapsöl. Bevorzugt stammen die nicht verseifbaren Lipide aus Canola-Öl.

Nicht verseifbare Lipide sind entweder Fettsäuren bzw. deren Derivate oder Isoprenderivate oder Triterpene. Sie sind beispielsweise aus Canola-Öl als leicht herzustellende Fraktion gewinnbar und sind überraschender Weise ebenfalls sehr gut zur Pflege der Haut und/oder zur Verhinderung des Austrocknens der Haut geeignet.

Bevorzugt ist daher auch ein erfindungsgemäßes Garn oder eine erfindungsgemäße textile Fläche, wobei die Polymerzusammensetzung Canola-Öl und/oder nicht verseifbare Lipide aus Canola-Öl enthält.

Bevorzugt ist auch ein erfindungsgemäßes Garn oder eine erfindungsgemäße textile Fläche, wobei die Polymerzusammensetzung Petrolatum, Sheabutter und ein Öl, das mindestens 25 Gew.-% Ölsäure enthält, enthält.

Bevorzugt ist auch ein erfindungsgemäßes Garn oder eine erfindungsgemäße textile Fläche, wobei die Polymerzusammensetzung Petrolatum, Sheabutter und Canola-Öl und/oder nicht verseifbare Lipide aus Canola-Öl enthält.

Bevorzugt ist auch ein erfindungsgemäßes Garn oder eine erfindungsgemäße textile Fläche, wobei die Polymerzusammensetzung Petrolatum, Sheabutter und ein Öl, ausgewählt aus der Gruppe bestehend aus Canola-Öl, Marula-Öl und Olivenöl enthält.

Bevorzugt ist auch ein erfindungsgemäßes Garn oder eine erfindungsgemäße textile Fläche, wobei die Polymerzusammensetzung Sheabutter und ein Öl, das mindestens 25 Gew.-% Ölsäure enthält, enthält.

Bevorzugt ist auch ein erfindungsgemäßes Garn oder eine erfindungsgemäße textile Fläche, wobei die Polymerzusammensetzung Sheabutter und ein Öl, ausgewählt aus der Gruppe bestehend aus Canola-Öl, Marula-Öl und Olivenöl enthält.

Bevorzugt ist daher auch ein erfindungsgemäßes Garn oder eine erfindungsgemäße textile Fläche, wobei die Polymerzusammensetzung Öl, das mindestens 25 Gew.-% Ölsäure enthält, und einen weiteren Wirkstoff enthält, ausgewählt aus der Gruppe bestehend aus Dicaprylyl Carbonat, Cetylisononanoat, Isoprpopyl Isostearat, Isostearyl Isostearat und Mischungen davon.

Bevorzugt ist daher auch ein erfindungsgemäßes Garn oder eine erfindungsgemäße textile Fläche, wobei die Polymerzusammensetzung Öl, das mindestens 25 Gew.-% Ölsäure enthält, und einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist daher auch ein erfindungsgemäßes Garn oder eine erfindungsgemäße textile Fläche, wobei die Polymerzusammensetzung Marula-Öl, Olivenöl und/oder Rapsöl und einen weiteren Wirkstoff enthält, ausgewählt aus der Gruppe bestehend aus Dicaprylyl Carbonat, Cetylisononanoat, Isoprpopyl Isostearat, Isostearyl Isostearat und Mischungen davon.

Bevorzugt ist daher auch ein erfindungsgemäßes Garn oder eine erfindungsgemäße textile Fläche, wobei die Polymerzusammensetzung Marula-Öl und einen weiteren Wirkstoff enthält, ausgewählt aus der Gruppe bestehend aus Dicaprylyl Carbonat, Cetylisononanoat, Isoprpopyl Isostearat, Isostearyl Isostearat und Mischungen davon.

Bevorzugt ist daher auch ein erfindungsgemäßes Garn oder eine erfindungsgemäße textile Fläche, wobei die Polymerzusammensetzung Marula-Öl, Olivenöl und/oder Rapsöl und einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist daher auch ein erfindungsgemäßes Garn oder eine erfindungsgemäße textile Fläche, wobei die Polymerzusammensetzung Marula-Öl und einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist daher auch ein erfindungsgemäßes Garn oder eine erfindungsgemäße textile Fläche, wobei die Polymerzusammensetzung a) Petrolatum und b) Öl, das mindestens 25 Gew.-% Ölsäure enthält.

Bevorzugt ist daher auch ein erfindungsgemäßes Garn oder eine erfindungsgemäße textile Fläche, wobei die Polymerzusammensetzung a) Petrolatum und b) Marula-Öl, Olivenöl und/oder Rapsöl, insbesondere Canola-Öl, enthält.

Bevorzugt ist daher auch ein erfindungsgemäßes Garn oder eine erfindungsgemäße textile Fläche, wobei die Polymerzusammensetzung Petrolatum und Marula-Öl enthält.

Bevorzugt ist daher auch ein erfindungsgemäßes Garn oder eine erfindungsgemäße textile Fläche, wobei die Polymerzusammensetzung a) Petrolatum, b) Öl, das mindestens 25 Gew.-% Ölsäure enthält, und c) einen weiteren Wirkstoff enthält, ausgewählt aus der Gruppe bestehend aus Dicaprylyl Carbonat, Cetylisononanoat, Isoprpopyl Isostearat, Isostearyl Isostearat und Mischungen davon.

Bevorzugt ist daher auch ein erfindungsgemäßes Garn oder eine erfindungsgemäße textile Fläche, wobei die Polymerzusammensetzung a) Petrolatum, b) Marula-Öl, Olivenöl und/oder Rapsöl und c) einen weiteren Wirkstoff enthält, ausgewählt aus der Gruppe bestehend aus Dicaprylyl Carbonat, Cetylisononanoat, Isoprpopyl Isostearat, Isostearyl Isostearat und Mischungen davon.

Bevorzugt ist daher auch ein erfindungsgemäßes Garn oder eine erfindungsgemäße textile Fläche, wobei die Polymerzusammensetzung Petrolatum, Marula-Öl und einen weiteren Wirkstoff enthält, ausgewählt aus der Gruppe bestehend aus Dicaprylyl Carbonat, Cetylisononanoat, Isoprpopyl Isostearat, Isostearyl Isostearat und Mischungen davon. Bevorzugt ist daher auch ein erfindungsgemäßes Garn oder eine erfindungsgemäße textile Fläche, wobei die Polymerzusammensetzung a) Petrolatum, b) Öl, das mindestens 25 Gew.-% Ölsäure enthält, und c) einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist daher auch ein erfindungsgemäßes Garn oder eine erfindungsgemäße textile Fläche, wobei die Polymerzusammensetzung a) Petrolatum, b) Sheabutter und c) einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist daher auch ein erfindungsgemäßes Garn oder eine erfindungsgemäße textile Fläche, wobei die Polymerzusammensetzung a) Petrolatum, b) Marula-Öl, Olivenöl und/oder Rapsöl und c) einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Bevorzugt ist daher auch ein erfindungsgemäßes Garn oder eine erfindungsgemäße textile Fläche, wobei die Polymerzusammensetzung Petrolatum, Marula-Öl und einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

Die genannten Öle und auch Fette sind geeignete Partner für Petrolatum in einer bevorzugten lipophilen Mischung zur Additivierung von Fasern, insbesondere Fasern aus Biopolymeren, insbesondere Cellulosefasern. Deren Verarbeitung in Textilien und Medizintextilien führt nach deren Abgabe an das Stratum corneum beim Träger zum Hautfeuchte-Erhalt ("Skin Hydration") und Rauhigkeits-Abnahme der Haut und wirkt kontinuierlich über eine Zeitraum bis zu 28 Tage und länger. Die Wirksamkeit über einen solchen Zeitraum ist insbesondere bei medizinischen Hilfsmitteln wie Bandagen und Kompressions- und Stützstrümpfen vorteilhaft, da diese oftmals dauerhaft, also über einen längeren Zeitraum getragen werden.

Die Polymerzusammensetzung enthält also erfindungsgemäß neben dem Polymer zusätzlich mindestens einen Wirkstoff, wobei es sich bei dem Wirkstoff um Petrolatum und/oder einem Öl, wobei das Öl mindestens 25 Gew.-% Ölsäure enthält handeln kann, gemäß Anspruch 1. Natürlich sind also auch das Vorhandensein dieser Wirkstoffe und Mischungen dieser Wirkstoffe von der Erfindung erfasst.

Es zeigte sich überraschender Weise, dass solche Wirkstoffe in eine Polymerzusammensetzung eingebracht werden können und dort als Additiv ihre Wirkung, insbesondere direkt auf der anliegenden Haut entfalten können, wenn die Polymerzusammensetzung in Form von entsprechenden Produkten wie Kleidungsstücken und medizinischen Hilfsmitteln an der Haut anliegt. Insbesondere können diese Wirkstoffe dabei in vorteilhafter Weise ein Austrocknen der Haut verhindern. Auch können die Wirkstoffe dazu verwendet werden, weitere Wirkstoffe in die Haut eindringen zu lassen.

Es zeigte sich, dass insbesondere Polymerzusammensetzungen aus Biopolymeren, beispielsweise Cellulose, besonders gut mit Petrolatum und/oder einem hier genannten Öl oder Fett oder einem hier genannten nicht verseifbaren Lipid beladen werden können und dies in Gewichtsanteilen von insbesondere bis zu 25 % und mehr für Petrolatum und insbesondere bis zu 35 % und mehr für die Öle, bezogen auf Cellulose. Sehr gute Ergebnisse erreicht wurden beispielsweise mit Gewichtsanteilen von insbesondere bis zu 23 % für Petrolatum und insbesondere bis zu 11 % für die Öle, bezogen auf Cellulose.

Bevorzugt enthält die Polymerzusammensetzung das Petrolatum in einem Mengenanteil (bezogen auf das Gesamtgewicht der Polymerzusammensetzung) von mindestens 1 Gew.-%.

Bevorzugt enthält die Polymerzusammensetzung das Petrolatum in einem Mengenanteil (bezogen auf das Gesamtgewicht der Polymerzusammensetzung) von mindestens 5 Gew.-%.

Bevorzugt enthält die Polymerzusammensetzung das Petrolatum in einem Mengenanteil (bezogen auf das Gesamtgewicht der Polymerzusammensetzung) von mindestens 10 Gew.-%.

Bevorzugt enthält die Polymerzusammensetzung das Petrolatum in einem Mengenanteil (bezogen auf das Gesamtgewicht der Polymerzusammensetzung) von mindestens 1 Gew.-% und höchstens 40 Gew.-%, insbesondere höchstens 35 Gew.-%. Bevorzugt enthält die Polymerzusammensetzung das Petrolatum in einem Mengenanteil (bezogen auf das Gesamtgewicht der Polymerzusammensetzung) von höchstens 40 Gew.-%, insbesondere höchstens 35 Gew.-%.

Bevorzugt enthält die Polymerzusammensetzung das Petrolatum in einem Mengenanteil (bezogen auf das Gesamtgewicht der Polymerzusammensetzung) von mindestens 1 Gew.-% und höchstens 30 Gew.-%, insbesondere höchstens 25 Gew.-%. Bevorzugt enthält die Polymerzusammensetzung das Petrolatum in einem Mengenanteil (bezogen auf das Gesamtgewicht der Polymerzusammensetzung) von höchstens 30 Gew.-%, insbesondere höchstens 25 Gew.-%.

Bevorzugt enthält die Polymerzusammensetzung das Petrolatum in einem Mengenanteil (bezogen auf das Gesamtgewicht der Polymerzusammensetzung) von mindestens 1 Gew.-% und höchstens 23 Gew.-%. Bevorzugt enthält die Polymerzusammensetzung das Petrolatum in einem Mengenanteil (bezogen auf das Gesamtgewicht der Polymerzusammensetzung) von höchstens 23 Gew.-%.

Bevorzugt enthält die Polymerzusammensetzung das Öl in einem Mengenanteil (bezogen auf das Gesamtgewicht der Polymerzusammensetzung) von mindestens 1 Gew.-%.

Bevorzugt enthält die Polymerzusammensetzung das Öl in einem Mengenanteil (bezogen auf das Gesamtgewicht der Polymerzusammensetzung) von mindestens 5 Gew.-%.

Bevorzugt enthält die Polymerzusammensetzung das Öl in einem Mengenanteil (bezogen auf das Gesamtgewicht der Polymerzusammensetzung) von mindestens 1 Gew.-% und von höchstens 45 Gew.-%, insbesondere höchstens 40 Gew.-%. Bevorzugt enthält die Polymerzusammensetzung das Öl in einem Mengenanteil (bezogen auf das Gesamtgewicht der Polymerzusammensetzung) von höchstens 45 Gew.-%, insbesondere höchstens 40 Gew.-%.

Bevorzugt enthält die Polymerzusammensetzung das Öl in einem Mengenanteil (bezogen auf das Gesamtgewicht der Polymerzusammensetzung) von mindestens 1 Gew.-% und von höchstens 35 Gew.-%, insbesondere höchstens 20 Gew.-%. Bevorzugt enthält die Polymerzusammensetzung das Öl in einem Mengenanteil (bezogen auf das Gesamtgewicht der Polymerzusammensetzung) von höchstens 35 Gew.-%, insbesondere höchstens 20 Gew.-%.

Bevorzugt enthält die Polymerzusammensetzung das Öl in einem Mengenanteil (bezogen auf das Gesamtgewicht der Polymerzusammensetzung) von mindestens 1 Gew.-% und von höchstens 15 Gew.-%, insbesondere höchstens 12 Gew.-%. Bevorzugt enthält die Polymerzusammensetzung das Öl in einem Mengenanteil (bezogen auf das Gesamtgewicht der Polymerzusammensetzung) von höchstens 15 Gew.-%, insbesondere höchstens 12 Gew.-%.

Bevorzugt enthält die Polymerzusammensetzung das Öl in einem Mengenanteil (bezogen auf das Gesamtgewicht der Polymerzusammensetzung) von mindestens 1 Gew.-% und höchstens 11 Gew.-%. Bevorzugt enthält die Polymerzusammensetzung das Öl in einem Mengenanteil (bezogen auf das Gesamtgewicht der Polymerzusammensetzung) von höchstens 11 Gew.-%.

Ohne an die Theorie gebunden zu sein, sind insbesondere die bevorzugten Biopolymere, zum Beispiel Cellulose, in der Lage, Kavernen auszubilden, die als Beladungszonen für die Wirkstoffe dienen. Dabei ist in vorteilhafter Weise kein besonderes Herstellungsverfahren für die Biopolymere und die daraus hergestellten Fasern nötig.

Dabei konnte überraschender Weise auch festgestellt werden, dass die in der Polymerzusammensetzung als Additive enthaltene erfindungsgemäße Gruppe von Wirkstoffen über den Anwendungszeitraum eine gute Beständigkeit aufweist und somit über diesen Zeitraum ihre Wirkung entfalten kann.

Es zeigte sich darüber hinaus überraschenderweise, dass die erfindungsgemäßen Polymerzusammensetzungen, insbesondere, wenn sie Biopolymere enthalten, insbesondere Cellulose enthalten beziehungsweise Cellulose-basiert sind, zu Fasern führen, die trotz des Zusatzes mindestens einem der erfindungsgemäßen Additive dieselben hervorragenden Eigenschaften zeigen, wie reine Fasern, insbesondere Cellulosefasern, beispielsweise bezüglich ihrer Feinheit, Reißkraft, Reißkraftvariation, Dehnung, Nassdehnung, feinheitsbezogenen Reißkraft, feinheitsbezogenen Nassreißkraft, feinheitsbezogenen Schlingenreißkraft, Nassscheuerungsvariation und Nassmodulen. Dennoch zeigten diese Fasern gleichzeitig die durch das mindestens eine Additiv verliehenen positiven Eigenschaften.

Bevorzugt werden die Fasern aus einer Spinnlösung hergestellt. Es zeigte sich, dass trotz der Zugabe mindestens eines erfindungsgemäßen Additivs überraschenderweise keine Zersetzung einer Spinnlösung, insbesondere, wenn sie Cellulose enthält, und ganz besonders wenn die Spinnlösung aus Cellulose, N-Methylmorpholin-N-Oxid (NMMNO) und Wasser als Hauptkomponenten gebildet wird.

Geeignete Herstellverfahren, insbesondere für Polymerzusammensetzungen, die Cellulose oder modifizierte Cellulose enthalten, und insbesondere in Form von Formkörpern sind aus der DE 10 2007 054 702 A1 bekannt.

Die Polymerzusammensetzung kann Polymere enthalten, wie sie typischer weise für die Herstellung von Fasern, Filamenten, Garnen, Vliesstoffen, Kleidungsstücken und medizinischen Hilfsmitteln wie Bandagen und Kompressionsstrümpfen verwendet werden.

Bevorzugt ist das Polymer ein Biopolymer, besonders bevorzugt ein technisches Biopolymer.

In einer bevorzugten Ausführungsform ist das Polymer ausgewählt aus der Gruppe bestehend aus Cellulose, modifizierte Cellulose, Polyamid, Polyacryl, Polyester, Polyurethan, Polyethylen, Polypropylen, Modifikationen davon und Mischungen davon. In einer bevorzugten Ausführungsform ist das Polymer ausgewählt aus der Gruppe bestehend aus Cellulose, modifizierte Cellulose, Polyamid, Polyacryl, Polyester, Modifikationen davon und Mischungen davon.

In einer bevorzugten Ausführungsform ist das Polymer ausgewählt aus der Gruppe bestehend aus Cellulose, modifizierte Cellulose, Viskose, Polylactiden, Modifikationen davon, Derivaten davon und Mischungen davon.

In einer bevorzugten Ausführungsform ist das Polymer Cellulose, modifizierte Cellulose, Viskose oder Polylactid. In einer bevorzugten Ausführungsform ist das Polymer Cellulose oder Polylactid.

In einer alternativen Ausführungsform ist das Polymer ein Stärkepolymer.

In einer bevorzugten Ausführungsform ist das Polymer biologisch abbaubar. Das biologisch abbaubare Polymer ist bevorzugt ausgewählt aus der Gruppe bestehend aus Cellulose, modifizierter Cellulose, Latex, Eiweiß pflanzlicher sowie tierischer Herkunft sowie Gemischen davon. Auch können Polykondensations- und Polymerisations-Polymere, Polyurethane, Polyester, Polyacryle und Gemische von diesen Materialien verwendet werden.

In einer alternativen Ausführungsform kann jedoch auch vorgesehen sein, dass die erfindungsgemäße Polymerzusammensetzung auch nicht-biologisch abbaubare Polymere enthält.

Beispiele solcher Polymere sind Polyamide, aromatische Polyamide, insbesondere Aramide, Polyacrylnitril, Polyester oder Polyvinylalkohole.

In einer bevorzugten Ausführungsform ist das Polymer Cellulose, modifizierte Cellulose, oder Modifikationen davon und Mischungen davon. In einer bevorzugten Ausführungsform ist das Polymer Cellulose oder modifizierte Cellulose.

Bei Cellulose handelt es sich um ein ein hydrophiles Netzwerk ausbildendes Polymer, dessen supramolekulare Strukturen sowohl in Lösung als auch im Festkörper über ebenfalls hydrophile Wasserstoff-Brückenbindungen stabilisiert sind. Dagegen sind die in der vorliegenden Erfindung verwendeten Wirkstoffe ausgewählt aus der Gruppe bestehend aus eine innere Okklusion erzeugende Wirkstoffe, eine äußere Okklusion erzeugende Wirkstoffe und Feuchtigkeitsspendende Wirkstoffe eher unpolare, lipophile organische Verbindungen. Es zeigte sich nun im Rahmen der vorliegenden Erfindung überraschenderweise, dass sich solche unpolaren, lipophilen Wirkstoffe in die cellulosischen Filamente und Fasern einbringen lassen. Ebenso überraschend ist es, dass die in dem hydrophilen Cellulosenetzwerk inkorporierten und lipophilen organischen Wirkstoffe sich derart homogen und mikropartikulär verteilen lassen, dass eine feindosierbare und gleichmäßige Speicherung sowie kontrollierte Freisetzung dieser unpolaren lipophilen organischen Wirkstoffe aus den Fasern ermöglicht wird.

Bevorzugt wird die erfindungsgemäße Polymerzusammensetzung aus einer Spinnlösung hergestellt. Bevorzugt liegen die Wirkstoffe in der Polymerzusammensetzung als Mikroeinschlüsse vor.

Unter modifizierter Cellulose wird insbesondere Carboxyethyl-Cellulose, Methyl-Cellulose, Nitrat-Cellulose, Kupfer-Cellulose, Viskose, also Cellulose-Xanthoghenat, Cellulosecarbamat und Celluloseacetat verstanden.

In einer alternativen Ausführungsform ist das Polymer ein Polyamid, ein Polyacryl, ein Polyester oder Modifikationen davon oder ein Derivat davon und Mischungen davon. In einer bevorzugten Ausführungsform ist das Polymer ein Polyamid, oder Modifikationen davon. In einer bevorzugten Ausführungsform ist das Polymer ein Polyacryl oder Modifikationen davon. In einer bevorzugten Ausführungsform ist das Polymer ein Polyester oder Modifikationen davon.

In einer bevorzugten Ausführungsform ist das Polymer ein Polyamid oder ein Derivat davon. In einer bevorzugten Ausführungsform ist das Polymer ein Polyacryl oder ein Derivat davon. In einer bevorzugten Ausführungsform ist das Polymer ein Polyester oder ein Derivat davon.

Beispiele für Polykondensations- und Polymerisations-Polymere sind Polyamide, die zum Beispiel mit Methyl-, Hydroxi-, oder Benzylgruppen substituiert sind. Beispiele für Polyurethane sind solche, die auf der Basis von Polyether-Polyolen aufgebaut sind.

In einer alternativen Ausführungsform ist das Polymer ein Polylactid oder eine Modifikationen davon oder ein Derivaten davon. In einer alternativen Ausführungsform ist das Polymer ein Polylactid.

Bevorzugt ist der mindestens eine Wirkstoff in die Polymerzusammensetzung inkorporiert und insbesondere nicht durch Tränkung oder Beschichtung auf die Trägermatrix aufgetragen.

Wie sich aus der vorangegangenen Beschreibung ergibt, können die Fasern des erfindungsgemäßen Garns oder der erfindungsgemäßen textilen Fläche, jegliche Kombination der genannten bevorzugten Ausführungsformen der Polymere und der genannten bevorzugten Ausführungsformen der genannten Wirkstoffe, insbesondere des Petrolatums, der Fette, der Öle, der nicht verseifbaren Lipide, aber auch der anderen Wirkstoffe, aufweisen.

Die vorliegende Erfindung offenbart auch einen Formkörper oder eine Vielzahl von Formkörpern umfassend eine erfindungsgemäße Polymerzusammensetzung.

Ein Vorteil der Formkörper, in denen die erfindungsgemäßen Additive enthalten sind, ist der gleichmäßige Einbau der Wirkstoffe in dem Formkörper, insbesondere in die Fasermatrix, bei unterschiedlich herstellbaren Faserquerschnitten. Dabei ist auch die Verarbeitung als Monofilament- oder Endlosfilamentgarn möglich, wodurch sich eine Einsatzmöglichkeit bei technischen Artikeln ergibt.

Die Formkörper können für verschiedenste Zwecke verwandt werden.

Bevorzugt ist der Formkörper eine Faser. Bevorzugt ist die Faser eine Stapelfaser, ein Monofilament, ein multifiles Filament oder ein multifiles Endlosfilament.

Bevorzugt ist also ein erfindungsgemäßes Garn oder eine erfindungsgemäße textile Fläche, wobei die Fasern Stapelfasern, Monofilamente, multifile Filamente oder multifile Endlosfilamente sind.

Insbesondere wenn die Faser eine cellulosische Faser ist, kann sie beispielsweise wie in den Figuren 1 und 2 der DE 10 2007 054 702 A1 gezeigt aufgebaut sein. Der mindestens eine Wirkstoff kann in Form von dispergierten Einschlüssen in einer Matrix, insbesondere Cellulosematrix vorliegen.

Bevorzugt ist der mindestens eine Wirkstoff mit zumindest einem hydrophoben Verdickungsmittel stabilisiert.

Insbesondere, wenn aus den Fasern eine erfindungsgemäße textile Fläche hergestellt wird, kann dieses nicht nur aus der erfindungsgemäßen Faser bestehen, sondern auch zusätzliche Komponenten enthalten, beispielsweise Baumwolle, Lyocell, Rayon, Carbacell, Polyester, Polyamid, Celluloseacetat, Acrylat, Polypropylen oder Gemische davon.

Die vorliegende Erfindung betrifft auch die Verwendung eines erfindungsgemäßen Formkörpers zur Herstellung von Garnen.

Die vorliegende Erfindung betrifft auch ein Garn, enthaltend einen Formkörper nach Anspruch 1. Die vorliegende Erfindung betrifft auch ein Garn, enthaltend die erfindungsgemäßen Formkörper als eine Grundkomponente. Die vorliegende Erfindung betrifft auch ein Garn, bestehend aus erfindungsgemäßen Formkörpern.

Die vorliegende Erfindung betrifft auch einen Vliesstoff, enthaltend ein erfindungsgemäßes Garn.

Bevorzugt ist also ein erfindungsgemäßes Garn oder eine erfindungsgemäße textile Fläche, wobei die Fasern in Form von Garn vorliegen, oder wobei die textile Fläche ein Vliesstoff oder ein Filz ist.

Bevorzugt ist eine erfindungsgemäße textile Fläche, wobei die Fasern in Form von Garn vorliegen.

Bevorzugt ist die textile Fläche ein Vliesstoff oder ein Filz. Bevorzugt ist die textile Fläche ein Vliesstoff. Bevorzugt ist die textile Fläche ein Filz.

Bevorzugt ist also ein erfindungsgemäßes Garn oder eine erfindungsgemäße textile Fläche, wobei die textile Fläche ein Gestrick, ein Gewirk oder ein Gewebe ist.

Bevorzugt ist die textile Fläche ein Gestrick, ein Gewirk oder ein Gewebe. Bevorzugt ist die textile Fläche ein Gestrick. Bevorzugt ist die textile Fläche ein Gewirk. Bevorzugt ist die textile Fläche ein Gewebe. Bevorzugt ist die textile Fläche ein Gestrick oder ein Gewirk. Bevorzugt ist die textile Fläche eine Maschenware.

Die vorliegende Erfindung betrifft auch Kleidungstück, Sporthilfsmittel oder medizinisches Hilfsmittel, enthaltend ein erfindungsgemäßes Garn oder eine erfindungsgemäße textile Fläche.

Die vorliegende Erfindung betrifft auch Kleidungstück, Sporthilfsmittel oder medizinisches Hilfsmittel, enthaltend ein erfindungsgemäßes Garn oder eine erfindungsgemäße textile Fläche.

Bevorzugt handelt es sich bei dem Kleidungsstück oder Sporthilfsmittel um ein Trikot, eine Sporthose, eine Unterhose, ein Unterhemd oder einen Strumpf. Alternativ kann es sich bei dem Kleidungsstück auch um einen Handschuh handeln.

Bevorzugt handelt es sich bei dem medizinischen Hilfsmittel um eine Bandage oder einen Kompressionsstrumpf. Bevorzugt handelt es sich bei dem medizinischen Hilfsmittel um eine Bandage. Bevorzugt handelt es sich bei dem medizinischen Hilfsmittel um einen Kompressionsstrumpf.

Die vorliegende Erfindung betrifft auch ein Kleidungstück oder ein medizinisches Hilfsmittel, enthaltend ein erfindungsgemäßes Garn. Bevorzugt handelt es sich um Kleidungsstücke oder medizinische Hilfsmittel, die beim Tragen zumindest teilweise direkt an der Haut anliegen.

Die vorliegende Erfindung betrifft auch ein Kleidungstück, enthaltend ein erfindungsgemäßes Garn. Bevorzugt ist das Kleidungsstück Unterwäsche, insbesondere eine Unterhose, ein Unterhemd oder ein Strumpf. Alternativ kann es sich bei dem Kleidungsstück auch um einen Handschuh handeln.

Die vorliegende Erfindung betrifft auch ein medizinisches Hilfsmittel, enthaltend ein erfindungsgemäßes Garn. Bevorzugt handelt es sich bei dem medizinischen Hilfsmittel um einen Kompressionsstrumpf oder um eine Bandage.

Die vorliegende Erfindung betrifft auch eine Sportbandage und andere Sporthilfsmittel, enthaltend ein erfindungsgemäßes Garn. Die vorliegende Erfindung betrifft auch eine Sportbandage und andere Sporthilfsmittel, enthaltend eine erfindungsgemäße textile Fläche.

Die vorliegende Erfindung offenbart auch die Verwendung einer offenbarten Polymerzusammensetzung oder eines offenbarten Formkörpers in einem Kleidungstück, einem Sporthilfsmittel oder in einem medizinischen Hilfsmittel zur Pflege der Haut.

Die vorliegende Erfindung offenbart auch die Verwendung einer offenbarten Polymerzusammensetzung oder eines offenbarten Formkörpers in einem Kleidungstück, einem Sporthilfsmittel oder in einem medizinischen Hilfsmittel zur Verhinderung des Austrocknens der Haut.

Die vorliegende Erfindung betrifft auch die Verwendung von Fasern zur Herstellung von Garnen oder textilen Flächen, insbesondere Filze oder Vliesen, wobei die Fasern eine Polymerzusammensetzung umfassen, enthaltend ein Polymer und mindestens einen Wirkstoff ausgewählt aus der Gruppe bestehend aus Petrolatum, einem Öl, wobei das Öl mindestens 25 Gew.-% Ölsäure enthält, einem nicht verseifbaren Lipid aus einem Öl, wobei das Öl mindestens 25 Gew.-% Ölsäure enthält, Sheabutter und Mischungen davon, gemäß Anspruch 14.

Die vorliegende Erfindung betrifft auch die Verwendung eines erfindungsgemäßen Garns oder einer erfindungsgemäßen textilen Fläche in einem Kleidungstück, in einem Sporthilfsmittel oder in einem medizinischen Hilfsmittel zur Pflege der Haut und/oder zur Verhinderung des Austrocknens der Haut.

## Patentansprüche

1. Garn oder textile Fläche, enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl oder einem Fett, wobei das Öl oder das Fett mindestens 25 Gew.-% Ölsäure enthält, einem nicht verseifbaren Lipid aus einem Öl, wobei das Öl mindestens 25 Gew.-% Ölsäure enthält, und Mischungen davon, **dadurch gekennzeichnet, dass** die Polymerzusammensetzung einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

2. Garn oder textile Fläche nach einem der vorhergehenden Ansprüche, wobei das Polymer ein Biopolymer ist.

3. Garn oder textile Fläche nach einem der vorhergehenden Ansprüche, wobei das Polymer ausgewählt ist aus der Gruppe bestehend aus Cellulose, modifizierte Cellulose, Viskose, Polylactiden, Modifikationen davon, Derivaten davon und Mischungen davon.

4. Garn oder textile Fläche nach einem der vorhergehenden Ansprüche, wobei der mindestens eine Wirkstoff Petrolatum ist.

5. Garn oder textile Fläche nach einem der vorhergehenden Ansprüche, wobei die Polymerzusammensetzung Petrolatum und einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

6. Garn oder textile Fläche nach einem der vorhergehenden Ansprüche, wobei der mindestens eine Wirkstoff zur Penetrationsverstärkung durch die Haut dient.

7. Garn oder textile Fläche nach einem der vorhergehenden Ansprüche, wobei der mindestens eine Wirkstoff ein eine Okklusion erzeugender Wirkstoff ist.

8. Garn oder textile Fläche nach einem der vorhergehenden Ansprüche, wobei die Fasern Stapelfasern, Monofilamente, multifile Filamente oder multifile Endlosfilamente sind.

9. Textile Fläche nach einem der vorhergehenden Ansprüche, wobei die Fasern in Form von Garn vorliegen, oder wobei die textile Fläche ein Vliesstoff oder ein Filz ist.

10. Textile Fläche nach einem der vorhergehenden Ansprüche, wobei die textile Fläche ein Gestrick, ein Gewirk oder ein Gewebe ist.

11. Kleidungstück, Sporthilfsmittel oder medizinisches Hilfsmittel, enthaltend einen Garn oder eine textile Fläche nach einem der Ansprüche 1 bis 10 oder enthaltend Fasern, wobei die Fasern eine Polymerzusammensetzung umfassen, enthaltend ein Polymer und mindestens einen Wirkstoff ausgewählt aus der Gruppe bestehend aus Petrolatum, einem Öl oder einem Fett, wobei das Öl oder das Fett mindestens 25 Gew.-% Ölsäure enthält, einem nicht verseifbaren Lipid aus einem Öl, wobei das Öl mindestens 25 Gew.-% Ölsäure enthält, und Mischungen davon.

12. Kleidungsstück oder Sporthilfsmittel nach Anspruch 11, wobei es sich um ein Trikot, eine Sporthose, eine Unterhose, ein Unterhemd oder einen Strumpf handelt.

13. Medizinisches Hilfsmittel nach Anspruch 11, wobei es sich um eine Bandage oder einen Kompressionsstrumpf handelt.

14. Verwendung von Fasern zur Herstellung von Garnen oder textilen Flächen, insbesondere Filze oder Vliesen, wobei die Fasern eine Polymerzusammensetzung umfassen, enthaltend ein Polymer und mindestens einen Wirkstoff ausgewählt aus der Gruppe bestehend aus Petrolatum, einem Öl oder Fett, wobei das Öl oder das Fett mindestens 25 Gew.-% Ölsäure enthält, einem nicht verseifbaren Lipid aus einem Öl, wobei das Öl mindestens 25 Gew.-% Ölsäure enthält, und Mischungen davon, wobei die Polymerzusammensetzung einen weiteren Wirkstoff enthält, ausgewählt aus der Gruppe bestehend aus Dicaprylyl Carbonat, Cetylisononanoat, Cetearyl Isononanoat, Isopropyl Isostearat, Isostearyl Isostearat, Isopropyl Palmitat und Mischungen davon.

15. Verwendung eines Garns oder einer textilen Fläche nach einem der Ansprüche 1 bis 10 in einem Kleidungstück, in einem Sporthilfsmittel oder in einem medizinischen Hilfsmittel zur Pflege der Haut und/oder zur Verhinderung des Austrocknens der Haut.

## Claims

1. A thread or textile surface comprising fibres, wherein the fibres comprise a polymer composition containing a polymer and at least one agent selected from the group consisting of petrolatum, an oil or a fat, wherein the oil or the fat contains at least 25 wt% oleic acid, a non-saponifiable lipid from an oil, wherein the oil contains at least 25 wt% oleic acid, and mixtures thereof, **characterized in that** the polymer composition contains another agent selected from the group consisting of dicaprylyl carbonate, cetyl isononanoate, cetearyl isononanoate, isopropyl isostearate, isostearyl isostearate, isopropyl palmitate, and mixtures thereof.

2. The thread or textile surface according to any one of the preceding claims, wherein the polymer is a biopolymer.

3. The thread or textile surface according to any one of the preceding claims, wherein the polymer is selected from the group consisting of cellulose, modified cellulose, viscose, polylactides, modifications thereof, derivatives thereof, and mixtures thereof.

4. The thread or textile surface according to any one of the preceding claims, wherein the at least one agent is petrolatum.

5. The thread or textile surface according to any one of the preceding claims, wherein the polymer composition contains petrolatum and another agent selected from the group consisting of dicaprylyl carbonate, cetyl isononanoate, cetearyl isononanoate, isopropyl isostearate, isostearyl isostearate, isopropyl palmitate, and mixtures thereof.

6. The thread or textile surface according to any one of the preceding claims, wherein the at least one agent serves as a skin penetration enhancer.

7. The thread or textile surface according to any one of the preceding claims, wherein the at least one agent is an occlusive agent.

8. The thread or textile surface according to any one of the preceding claims, wherein the fibres are staple fibres, monofilaments, multifilaments, or continuous multifilaments.

9. The textile surface according to any one of the preceding claims, wherein the fibres are present in the form of thread, or wherein the textile surface is a nonwoven material or a felt.

10. The textile surface according to any one of the preceding claims, wherein the textile surface is a knitted fabric, a mesh fabric, or a woven fabric.

11. A clothing article, sports aid, or medical aid containing a thread or a textile surface according to any one of claims 1 to 10 or containing fibres, wherein the fibres comprise a polymer composition containing a polymer and at least one agent selected from the group consisting of petrolatum, an oil or a fat, wherein the oil or the fat contains at least 25 wt% oleic acid, a non-saponifiable lipid from an oil, wherein the oil contains at least 25 wt% oleic acid, and mixtures thereof.

12. The clothing article or sports aid according to claim 11, wherein this item is a jersey, sports pants, underpants, an undershirt, or a stocking.

13. The medical aid according to claim 11, wherein this item is a bandage or a compression stocking.

14. A use of fibres for producing threads or textile surfaces, in particular felts or nonwovens, wherein the fibres comprise a polymer composition containing a polymer and at least one agent selected from the group consisting of petrolatum, an oil or a fat, wherein the oil or the fat contains at least 25 wt% oleic acid, a non-saponifiable lipid from an oil, wherein the oil contains at least 25 wt% oleic acid, and mixtures thereof, wherein the polymer composition contains another agent selected from the group consisting of dicaprylyl carbonate, cetyl isononanoate, cetearyl isononanoate, isopropyl isostearate, isostearyl isostearate, isopropyl palmitate, and mixtures thereof.

15. A use of a thread or textile surface according to any one of claims 1 to 10 in a clothing article, in a sports aid, or in a medical aid for skin care and/or for preventing the skin from drying out.

## Revendications

1. Fil ou surface textile contenant des fibres, les fibres comprenant une composition polymère contenant un polymère et au moins un ingrédient actif choisi dans le groupe constitué par le pétrolatum, une huile ou une graisse, l'huile ou la graisse contenant au moins 25% en poids d'acide oléique, un lipide non saponifiable provenant d'une huile, l'huile contenant au moins 25% en poids d'acide oléique, et leurs mélanges , **caractérisée en ce que** la composition polymère contient un autre ingrédient actif choisi dans le groupe constitué par le carbonate de dicaprylyle, l'isononanoate de cétyle, l'isononanoate de cétéaryle, l'isostéarate d'isopropyle, l'isostéarate d'isostéaryle, le palmitate d'isopropyle et leurs mélanges.

2. Fil ou surface textile selon l'une quelconque des revendications précédentes, dans lequel le polymère est un biopolymère.

3. Fil ou surface textile selon l'une quelconque des revendications précédentes, dans lequel le polymère est choisi dans le groupe constitué par la cellulose, la cellulose modifiée, la viscose, les polylactides, leurs modifications, leurs dérivés et leurs mélanges.

4. Fil ou surface textile selon l'une quelconque des revendications précédentes, dans lequel le au moins un ingrédient actif est le pétrolatum.

5. Fil ou surface textile selon l'une quelconque des revendications précédentes, dans laquelle la composition polymère comprend du pétrolatum et un autre ingrédient actif choisi dans le groupe constitué par le carbonate de dicaprylyle, l'isononanoate de cétyle, l'isononanoate de cétéaryle, l'isostéarate d'isopropyle, l'isostéarate d'isostéaryle, le palmitate d'isopropyle et leurs mélanges.

6. Fil ou surface textile selon l'une quelconque des revendications précédentes, dans laquelle le au moins un ingrédient actif sert à améliorer la pénétration à travers la peau.

7. Fil ou surface textile selon l'une quelconque des revendications précédentes, dans laquelle le au moins un agent actif est un agent produisant une occlusion.

8. Fil ou surface textile selon l'une quelconque des revendications précédentes, dans lequel les fibres sont des fibres discontinues, des monofilaments, des filaments multifilaments ou des filaments continus multifilaments.

9. Surface textile selon l'une quelconque des revendications précédentes, dans laquelle les fibres sont sous forme de fil, ou dans laquelle la surface textile est un tissu non tissé ou un feutre.

10. Surface textile selon l'une quelconque des revendications précédentes, dans laquelle la surface textile est un tissu tricoté, un tissu à mailles ou un tissu tissé.

11. Vêtement, aide sportive ou aide médicale comprenant un fil ou une surface textile selon l'une quelconque des revendications 1 à 10 ou contenant des fibres, dans lequel les fibres comprenant une composition polymère contenant un polymère et au moins un ingrédient actif choisi dans le groupe constitué par le pétrolatum, une huile ou une graisse, l'huile ou la graisse contenant au moins 25% en poids d'acide oléique, un lipide non saponifiable provenant d'une huile, l'huile contenant au moins 25% en poids d'acide oléique, et leurs mélanges.

12. Vêtement ou aide sportive selon la revendication 11, dans lequel le vêtement ou aide sportif est un maillot, un short de sport, un pantalon, un gilet ou un bas.

13. Aide médicale selon la revendication 11, dans laquelle l'aide médicale est un bandage ou un bas de compression.

14. Utilisation de fibres pour la production de fils ou de surfaces textiles, en particulier de feutres ou de non tissés, les fibres comprenant une composition polymère contenant un polymère et au moins un ingrédient actif choisi dans le groupe constitué par le pétrolatum, une huile ou une graisse, l'huile ou la graisse contenant au moins 25% en poids d'acide oléique, un lipide non saponifiable provenant d'une huile, l'huile contenant au moins 25% en poids d'acide oléique, et leurs mélanges , la composition polymère contenant un autre ingrédient actif choisi dans le groupe constitué par le carbonate de dicaprylyle, l'isononanoate de cétyle, l'isononanoate de cétéaryle, l'isostéarate d'isopropyle, l'isostéarate d'isostéaryle, le palmitate d'isopropyle et leurs mélanges.

15. Utilisation d'un fil ou d'une surface textile selon l'une quelconque des revendications 1 à 10 dans un vêtement, dans une aide sportive ou dans une aide médicale pour le soin de la peau et/ou pour prévenir le dessèchement de la peau.
